# EUROPEAN PATENT APPLICATION

(11) **EP 0 900 546 A1**
(43) Date of publication of application: **10.03.1999**
(21) Application number: 98307087.1
(22) Date of filing: 03.09.1998
(51) Int. Cl.: A61B 17/34, A61M 5/32

(54) **Needle-catheter assembly for medical or veterinarian use**

(30) Priority: 05.09.1997 AR 10407297
(71) Applicant: Vega, Irene Cristina Luis, 1426 Buenos Aires (AR)
(72) Inventor: Vega, Irene Cristina Luis, 1426 Buenos Aires (AR)
(74) Representative: Style, Kelda Camilla Karen

(57) **Abstract**

A needle-catheter assembly (1) for medical or veterinarian use, particularly useful for gaining access into the medullary channel for therapeutic, anaesthetic or diagnostic purposes, comprises a body (2) made of a flexible but strong material, one of its ends having a conical tip (3) that is substantially solid and an inner hollow cylindrical length (5) with at least one opening proximal to said conical tip. The assembly also includes a rigid guide (7) so that it can be removably inserted into the cylindrical length of said body (2), said rigid guide (7) having a passage (9) that extends longitudinally along the same and being placed in front of the at least one opening in the body. The assembly enables the administration of a rachianaesthesia. The method of gaining access into the medullary channel is also disclosed. In a preferred embodiment, said method of gaining access into the medullary channel also includes the additional step of administering an anaesthetic.

## Description

The present invention relates to a needle - catheter assembly for medical or veterinarian use. More particularly it pertains to a needle - catheter assembly to be used when desiring to gain access to the inside of the medullary channel, for whatever purpose it might serve, such as, for example, for anesthetic, therapeutic or diagnostic purposes.

There are known the problems that arise when it is necessary to perform a rachianesthesia, that is the introduction of an anesthetic substance in the medulla channel. In early stages, a metal, beveled-tip needle was used, which was introduced between two vertebrae and pierced the dura mater to gain access to the spinal cord. It so happens that, when piercing the dura mater, the needle bevel cuts its fibers. When the withdrawal of medullary fluid is detected, a microcatheter having a distal outlet through which the anesthetic is going to flow is introduced through the needle. When the spinal cord is finally reached, the bevel-tipped needle is removed, letting the catheter remain inside the medullary channel.

However, this method has the drawback that this kind of bevel pointed needle cuts or breaks the dura mater fibers, thereby causing post-operative complications such as headaches.

In order to alleviate these difficulties, finer bevel-pointed needles have been developed, so as to attenuate the cutting of the dura mater fibers, although good results are not achieved with said needle, inasmuch as post-operative headaches are not generally avoided. Furthermore, as the catheter required is very thin, the following additional drawbacks are encountered:
- Inability to make the medullary fluid flow
- Bending of the catheter at the outlet of the needle
- Unsuitable anesthetic doses
- Catheter breakage
- Possible neurological damage
- Difficulty for introduction thereof into the spinal cord
- Difficulty for placement thereof.

In order to overcome these problems, mainly that of headaches, a cone-pointed metal needle, also known as "pencil point", has been developed. Said needle has a side opening through which the anesthetic is introduced. The use of this needle has the advantage that, on introducing the needle through the dura mater, the conic tip does not break the fibers therein, but separates them instead. However, this technique raises the issue of having to dispense the anesthetic as a single dose due to the fact that the "pencil point" needle has to be removed prior to surgery.

The "pencil point" needle also allows for the introduction of a very fine microcatheter, causing all of the aforementioned problems.

There are a considerable number of patents related to catheters of various characteristics, but in every case categorized under one of the concepts previously disclosed, i.e., a needle must be used prior to the insertion of a catheter.

By way of example, Argentine Patents Nos. 242,724; 242,906; 244,094; 245,003; 247,338 and 249,956 incorporate certain improvements, though none of them pertain or respond to the inventive concept of embodiments of the present invention.

Indeed, embodiments of this invention have been conceived in a totally different manner and it replaces and can exceed the results achieved by the needle-catheter assemblies of the prior art as mentioned above.

The needle-catheter assembly, or "soft needle" embodying the present invention is based on a novel concept, inasmuch as one element alone can include all that is necessary to effectively replace many of the known catheters. It has the advantage of being soft, while having enough rigidity to pierce the dura mater, reach the spinal cord and then become flexible, having all the features of a catheter and a pre-shaped curvature. Therefore, embodiments of this invention may be used to perform surgical anesthesia, intraoperative analgesia, postoperative analgesia, obstetric analgesia, and treatment for pain and infections and diagnostic procedures, among the most common applications.

Assemblies embodying this invention have various advantages, such as, to begin with, that it causes no postoperative headaches. Furthermore, the possibility of causing neurological damage is minimized, for it allows for the upward placement of the catheter, for the use of anesthetics at lower doses, lower concentrations and lower densities due to the presence of several side openings suitable for the passage of fluids therethrough. On the other hand, the possibility of rupturing the catheter is also minimized, since as it enters the spinal cord directly, its diameter may be greater than those normally used, and the material from which it can be built is soft but strong. Thus, its main feature is that it does not cause headaches and at the same time it is not difficult for it to gain access into the spinal cord.

It also has the significant advantage of allowing the administration of the appropriate amount of anesthesia in an only fractional and continuous dose, since in its preferred embodiment it has several side openings. Furthermore, it is possible to direct such catheter such that it enables the diffusion or permeation of the anesthetic into the spinal cord, by directing the end of the catheter toward the cephalic or caudal end (i.e. upwards or downwards), depending on the requirements and the drugs to be used. It also enables its use for analgesia during the postoperative period.

For a better understanding of the present invention and as to how the same may be carried into effect, reference will now be made by way of example to the accompanying drawings in which:

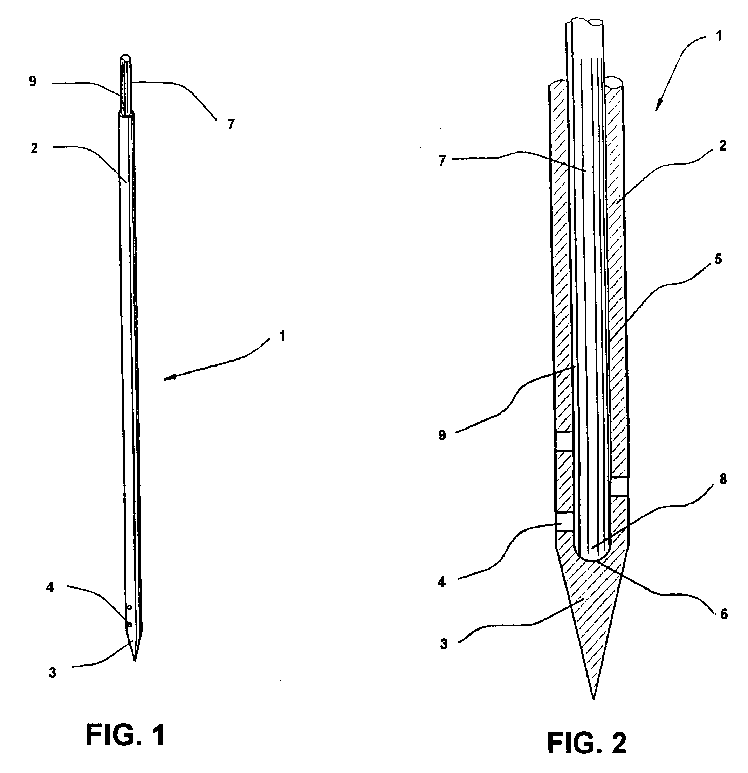

Figure 1 is a general perspective view of the needle - catheter assembly with its rigid guide partially inserted, whereas
Figure 2 shows a schematic view of a longitudinal section of the end portion of said element.

Thus, we can see that the needle - catheter assembly for medical use embodying the present invention, generally indicated by reference number 1, comprises a hollow cylindrical elongated body 2 in the shape of a soft sheath, which is usually built of a flexible material such as a Nylon, Teflon or polyamide-type material, or a similar material, having a solid tip 3. At least one through hole 4 is provided laterally (though it can have more than one openings as shown) communicating the outside of the soft sheath with the hollow inside or passage 5, which passage ends close to the conical tip, having an end surface 6 that, in a preferred embodiment, is concave to the hollow portion. A guide, (not shown) allows for the placement of the channel in front of the side opening of the "soft needle".

Complementing the needle - catheter assembly, there is a rigid guide 7 for its insertion, which must be built of a rigid material, preferably of a metallic material, the shape of which complements that of the inner passage 5 and is preferably cylindrical, ending in a "blunt" end 8. On its side, the guide has a longitudinal outer passage 9, which allows for the withdrawal of corporal fluids, particularly rachidian fluid, when the present device is inserted and it reaches into the spinal cord.

Therefore, when it is required to gain access into the medullary channel for any of the aforementioned purposes, an "inserting needle" is used, which needle is a known means, not shown in the previous drawings, the purpose of which is piercing the skin, passing between two vertebrae and piercing the yellow ligament. Thereafter, the needle 1 embodying the present invention with its rigid guide 7 is inserted through said inserting needle, and this assembly penetrates into the dura mater by means of its conical tip 3, thus gaining access to the medullary channel. When the withdrawal of fluids through the outer longitudinal channel 9 is validated, the rigid guide 7 is removed, the soft needle or catheter 1 is advanced and subsequently the inserting needle is also removed.

In the manner disclosed in the above paragraph, the entrance into the medullary channel is carried out in the absence of the inserting needle, for this needle is removed prior to the introduction of the assembly or soft needle with its guide. In an analogous way, when the above assembly goes through the dura mater, the inserting needle is removed and only the catheter - needle is left to work with, because once the rigid guide 7 has been removed, the inner passage 5 is free to let the fluid to be administered go through, which fluid leaves the device through the at least one through hole 4, though more openings can be provided if required.

Therefore, the "soft needle" thus becomes a catheter and it will act as such during surgery. When the metal guide is removed, the soft needle, now turned into a catheter, will assume the preshaped curvature within the medullary channel, said catheter being capable of being oriented as required depending on the surgical procedure to be performed.

Embodiments of this invention also pertain to a method of gaining access into the medullary channel of a mammal. Said method comprises the steps of inserting an inserting needle in the substeps of piercing the skin, passing between two vertebrae and piercing the yellow ligament, then proceeding to place the needle - catheter assembly 1 with its conical tip 3 with the help of its rigid guide 7 inside said inserting needle and, with this assembly, piercing the dura mater membrane by exerting the necessary pressure theron. On verifying that rachidian fluid is flowing through the outer longitudinal passage 9, the rigid guide is removed, for this means that the needle has been inserted into the medullary channel, so the soft needle or catheter 1 is then advanced, the inserting needle is removed and the catheter is suitably positioned into the medullary channel depending on the procedure to be executed.

In the case of a rachianesthesia, for example, once the above steps have been performed, an anesthetic is given. It is worth noting that the needle - catheter assembly thus placed allows to adjust the dose of the anesthetic according to the surgical procedure to be performed, making it possible to direct it toward the cephalic or caudal end and also allowing for a single, fractional or continuous dose. On the other hand, it can also be used for postoperative analgesia.

It will be understood that apparent modifications can be introduced on putting this invention into practice, without altering the spirit and scope thereof.

## Claims

1. A needle - catheter assembly for medical or veterinarian use, particularly useful for gaining access into the medullary channel for therapeutic, anesthetic or diagnostic purposes, characterized in that it comprises a body made of a flexible but strong material, one of its ends having a conical tip that is substantially solid and inside the body there is a hollow cylindrical length with at least one opening proximal to said conical tip; said assembly also including a rigid guide so that it can be removably inserted in the cylindrical length of said body, said rigid guide having a passage that extends longitudinally along the same and being placed in front of the at least one opening in the body.

2. The needle - catheter assembly of claim 1, characterized in that there are three openings.

3. The needle-catheter assembly of claim 1 or 2, characterized in that the openings are lateral.

4. The needle-catheter assembly of claim 1, 2 or 3, characterized in that the guide is made of a metal.

5. The needle-catheter assembly of claim 1, 2, 3 or 4, characterized in that it comprises a material selected among Nylon, Teflon and polyamide.

6. A method of gaining access into the medullary channel of a mammal, characterized in that it comprises the steps of introducing an inserting needle through the substeps of piercing the skin, passing between two vertebrae and penetrating into the yellow ligament, placing the needle -catheter assembly inside the inserting needle with its rigid guide inside and, with this assembly, piercing the dura mater, verifying that rachidian fluid is being withdrawn and removing the metal guide form the asssembly; manually advancing the soft needle or catheter, removing the inserting needle, and suitably positioning the catheter in the medullary channel.

7. A method according to claim 6, characterized in that it also comprises the step of administering an anaesthetic.

8. A method according to claim 7, characterized in that the anaesthetic is administered as a single, fractional or continuous dose.

9. A needle-catheter assembly for medical or veterinarian use, said assembly comprising a body made of a flexible but strong material, one of the ends of the body having a conical tip and inside the body there is a hollow cylindrical length with at least one opening proximal to said conical tip; and a guide removably insertable in the cylindrical length of said body, said guide having longitudinally extending passages.
